# EUROPEAN PATENT APPLICATION

(11) **EP 4 728 982 A1**
(43) Date of publication of application: **22.04.2026**
(21) Application number: 25202334.6
(22) Date of filing: 16.09.2025
(51) Int. Cl.: A61B 5/11, A61B 5/00, G06F 3/01

(54) **DETERMINATION METHOD, DETERMINATION DEVICE, DETERMINATION SYSTEM, CROSS REALITY DEVICE, TRAINING METHOD, TRAINING DEVICE, PROGRAM, AND STORAGE MEDIUM**

(30) Priority: 16.10.2024 JP 2024180991
(71) Applicant: KABUSHIKI KAISHA TOSHIBA, Kawasaki-shi, Kanagawa, 212-0013 (JP)
(72) Inventor: MASUI, Yukino, Kawasaki-shi, Kanagawa, 212-0013 (JP); NAMIOKA, Yasuo, Kawasaki-shi, Kanagawa, 212-0013 (JP); NAKAMURA, Hiroaki, Kawasaki-shi, Kanagawa, 212-0013 (JP); OSHIMA, Hirotomo, Kawasaki-shi, Kanagawa, 212-0013 (JP); HIRAHARA, Yoshiyuki, Kawasaki-shi, Kanagawa, 212-0013 (JP); YOSHII, Takanori, Kawasaki-shi, Kanagawa, 212-0013 (JP); FUKUDA, Masamitsu, Kawasaki-shi, Kanagawa, 212-0013 (JP); MITO, Yuya, Kawasaki-shi, Kanagawa, 212-0013 (JP)
(74) Representative: Hoffmann Eitle

(57) **Abstract**

According to one embodiment, a determination method causes a processing device to measure coordinates of a hand of a person based on an image in which the hand is visible. The determination method causes the processing device to receive a detected value from a sensor of a device held by the person. The determination method causes the processing device to input the coordinates and the detected value to an estimation model configured to estimate an action force of a body. The determination method causes the processing device to determine at least one selected from the group consisting of a load on the person, a danger to the person, and a proficiency of the person by using an action force of a body of the person output from the estimation model.

## Description

### FIELD

Embodiments of the invention generally relate to a determination method, a determination device, a determination system, a cross reality device, a training method, a training device, a program, and a storage medium.

### BACKGROUND

An excessive load that is applied to the body of a person in motion may hurt or injure the body. To improve the motion, it is effective to determine the proficiency of the person.

### BRIEF DESCRIPTION OF THE DRAWINGS

FIG. 1 is a flowchart showing a processing method according to an embodiment;
FIG. 2 is a schematic view showing a configuration of a determination system according to the embodiment;
FIG. 3 is a table illustrating data sets;
FIG. 4 is a table illustrating data sets;
FIG. 5 is a schematic view showing a training method of an estimation model;
FIG. 6 is a flowchart showing the training method of the estimation model;
FIG. 7 is a flowchart showing a training method of a danger determination model;
FIG. 8 is a schematic view showing the training method of the danger determination model;
FIG. 9 is a flowchart showing a training method of a proficiency determination model;
FIG. 10 is a schematic view showing the training method of the proficiency determination model;
FIG. 11 is a flowchart showing a determination method according to the embodiment;
FIG. 12 is a table illustrating an output result of a first estimation model;
FIG. 13 is a table illustrating thresholds for determining a load;
FIG. 14 is a flowchart illustrating a control of an output corresponding to the determination result of the load;
FIG. 15 is a schematic view showing an example of a cross reality device according to the embodiment;
FIG. 16 is a schematic view showing an output example of the determination system according to the embodiment;
FIG. 17 is a flowchart showing another determination method according to the embodiment;
FIG. 18 is a schematic view showing another output example of the determination system according to the embodiment;
FIG. 19 is a schematic view showing another example of the cross reality device according to the embodiment; and
FIG. 20 is a schematic view illustrating a hardware configuration.

### DETAILED DESCRIPTION

According to one embodiment, a determination method causes a processing device to measure coordinates of a hand of a person based on an image in which the hand is visible. The determination method causes the processing device to receive a detected value from a sensor of a device held by the person. The determination method causes the processing device to input the coordinates and the detected value to an estimation model configured to estimate an action force of a body. The determination method causes the processing device to determine at least one selected from the group consisting of a load on the person, a danger to the person, and a proficiency of the person by using an action force of a body of the person output from the estimation model.

Embodiments of the invention will now be described with reference to the drawings. The drawings are schematic or conceptual; and the relationships between the thicknesses and widths of portions, the proportions of sizes between portions, etc., are not necessarily the same as the actual values thereof. The dimensions and/or the proportions may be illustrated differently between the drawings, even in the case where the same portion is illustrated. In the drawings and the specification of the application, components similar to those described thereinabove are marked with like reference numerals, and a detailed description is omitted as appropriate.

When a person moves their body, forces act on the muscles, joints, and tendons of the body. When the forces acting on the body are large, there is a possibility that muscles or joints may be hurt, which may lead to serious injury. For example, the forces that are applied to joints have relationships with the forces acting on parts of the entire body when the person is in motion. Action forces of the body are represented by muscle activity levels, generated muscle forces, antagonistic muscle forces, joint forces, joint moments, etc. As an example, as the muscle activity level increases, muscles exert greater forces, and loads corresponding to the muscle force are applied to the joints. Therefore, to determine the load on a specific part of the body, it is effective to determine the action force of the body.

The action forces of the body also are dependent on how the person moves their body. For example, even when the same motion is performed, the action forces generated during the motions of an expert are different from the action forces generated during the motions of a beginner. Improving the motions of a beginner will lead to more efficient motions, prevention of bodily injuries, etc.

An aspect of an embodiment of the invention is directed to technology that can easily determine the load, danger, proficiency, etc., related to a task. Another aspect of the embodiment of the invention is directed to train a model to more easily estimate an action force of the body.

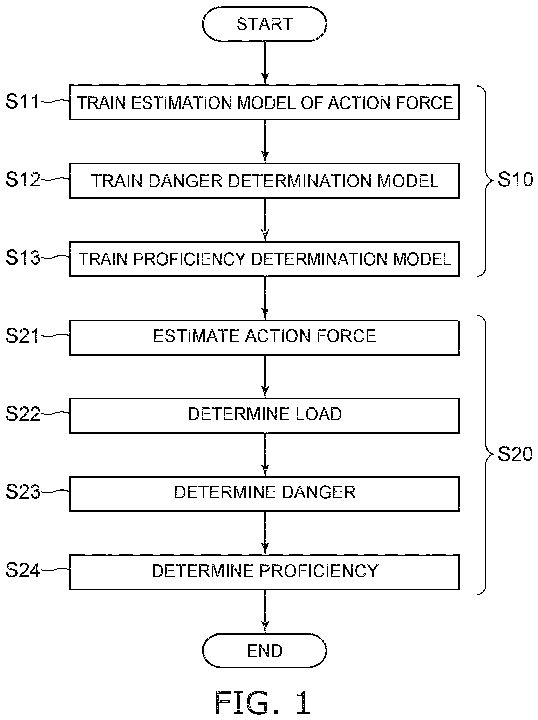

FIG. 1 is a flowchart showing a processing method according to the embodiment.

The processing method according to the embodiment mainly includes training (step S10) and determining (step S20). In the training (step S10), various data is used to train an estimation model that estimates an action force of a body (step S11). A danger determination model that determines a danger of a task also may be trained (step S12). A proficiency determination model that determines the proficiency of a worker may be trained (step S13). The sequence of steps S11 to S13 is modifiable.

In the determination (step S20), a trained estimation model is used to estimate the action force when a person actually moves their body (step S21). The load is determined by using the estimation result of the action force (step S22). The danger may be determined (step S23), and the proficiency may be determined (step S24). A trained danger determination model is used to determine the danger. A trained proficiency determination model is used to determine the proficiency. The sequence of steps S22 to S24 is modifiable.

One specific example of the processing will now be described. A case will be described where a person performs a screw-tightening task using a digital torque wrench.

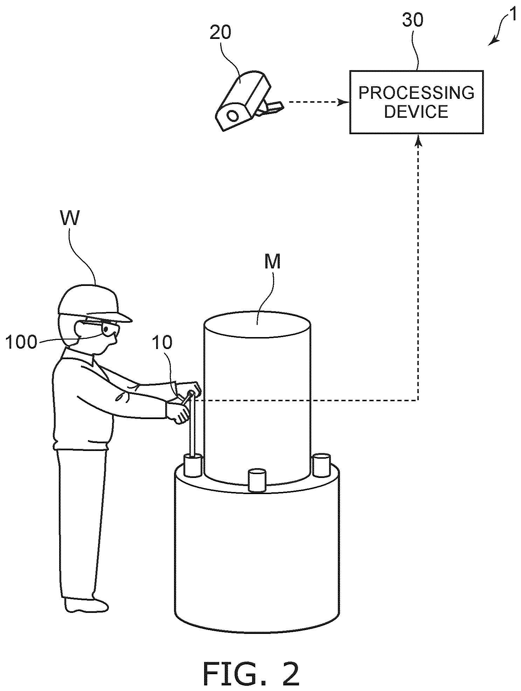

FIG. 2 is a schematic view showing a configuration of a determination system according to the embodiment.

As shown in FIG. 2, the determination system 1 according to the embodiment includes a digital torque wrench 10, an imaging device 20, and a processing device 30. In the illustrated example, a worker W performs screw-tightening at a member M by using the digital torque wrench 10. The digital torque wrench 10 can detect torque when a screw is turned. The digital torque wrench 10 is an example of a device that includes a sensor. The imaging device 20 images the worker W performing the task.

The processing device 30 receives the image of the worker W from the imaging device 20. The processing device 30 performs posture estimation of the worker W in the image. The posture estimation estimates the positions of joints of the worker W. A posture estimation model such as Dark Pose, Open Pose, or the like can be used for the posture estimation. For example, the posture estimation estimates the positions of the joints of the head, neck, shoulders, elbows, hands, fingers, lower back, knees, feet, etc. The processing device 30 calculates the coordinates of the joints based on the estimation result. The processing device 30 uses the coordinates of the joints to calculate angles of the joints by using inverse kinematics calculations.

The processing device 30 receives the detected torque from the digital torque wrench 10. The magnitude of the torque has a relationship with the force applied by the worker W to the digital torque wrench 10. In other words, the detected torque corresponds to the external force generated by the motion of the worker W. The processing device 30 uses the calculated coordinates of the joints and the detected torque to calculate the moments (the torques) of the joints by using inverse dynamics calculations. The processing device 30 also calculates action forces such as the muscle activity levels, generated muscle forces, antagonistic muscle forces, joint forces, etc., based on the calculated joint moments. An existing musculoskeletal simulation can be used to calculate the muscle activity levels, generated muscle forces, antagonistic muscle forces, joint forces, joint moments, etc.

The hand coordinates of the worker W are acquired separately from the action forces. The hand coordinates may be extracted from the results of the posture estimation. Or, in the example shown in FIG. 2, the worker W performs the task while wearing the mixed reality (MR) device 100. The MR device 100 can display various information to the worker W. The MR device 100 also includes an imaging device and can perform hand tracking. In hand tracking, the hands of the worker W are detected based on the image; and the hand coordinates of the worker W are calculated. The hand coordinates calculated by the MR device 100 may be referenced.

The digital torque wrench 10 continuously detects the torque. The imaging device 20 repeats the imaging. The processing device 30 repeats the calculation of the joint coordinates and the calculation of the action force. The MR device 100 repeatedly performs the hand tracking. Time-series data of the hand coordinates, time-series data of the torque, and time-series data of the action force are obtained thereby. Based on the time-series data, the processing device 30 generates a data set of the hand coordinates, torque, and action force at any timing. Data sets are prepared respectively for the hand coordinates, the torque, and the action force at multiple timings.

A case where the action force is mainly the muscle activity level will now be described.

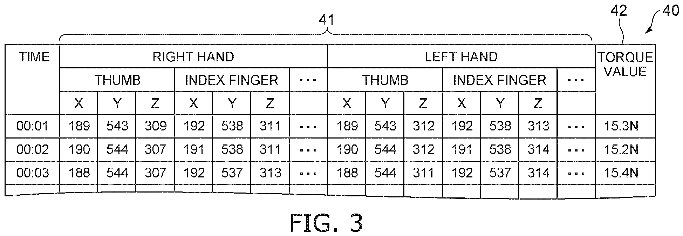

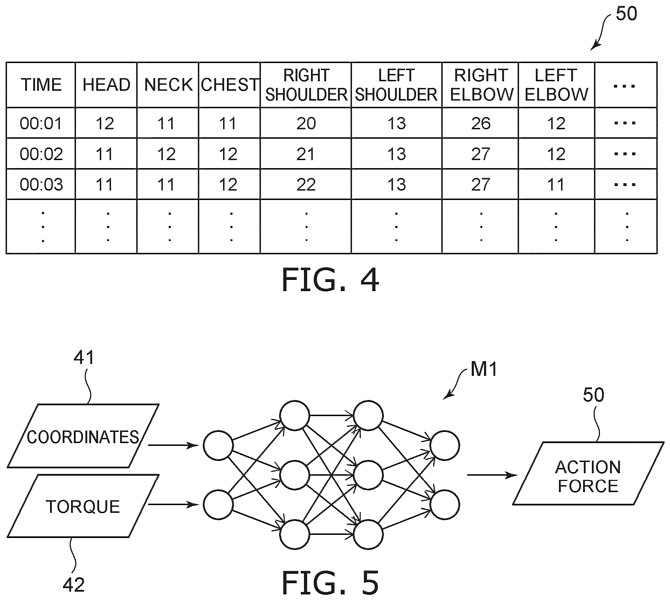

FIGS. 3 and 4 are tables illustrating data sets.

A table 40 shown in FIG. 3 includes hand coordinates 41 every second and a torque 42 every second. A table 50 shown in FIG. 4 shows the muscle activity levels of body parts every second. In other words, in the illustrated example, a data set is prepared for each second.

FIG. 5 is a schematic view showing a training method of an estimation model.

As shown in FIG. 5, the processing device 30 trains the estimation model M1 to estimate the action forces of the body parts. At this time, the hand coordinates and the torque are used as input data; and the action forces are used as output data. Supervised learning of the estimation model M1 is performed using multiple data sets that are prepared. As a result, the estimation model M1 is trained to output (estimate) action forces according to the input of the hand coordinates and the torque. To increase the estimation accuracy, it is favorable for the estimation model M1 to include a neural network. It is more favorable for the estimation model M1 to include a graph neural network (GNN) or a recurrent neural network (RNN). It is favorable for the RNN to have a long short-term memory (LSTM) structure.

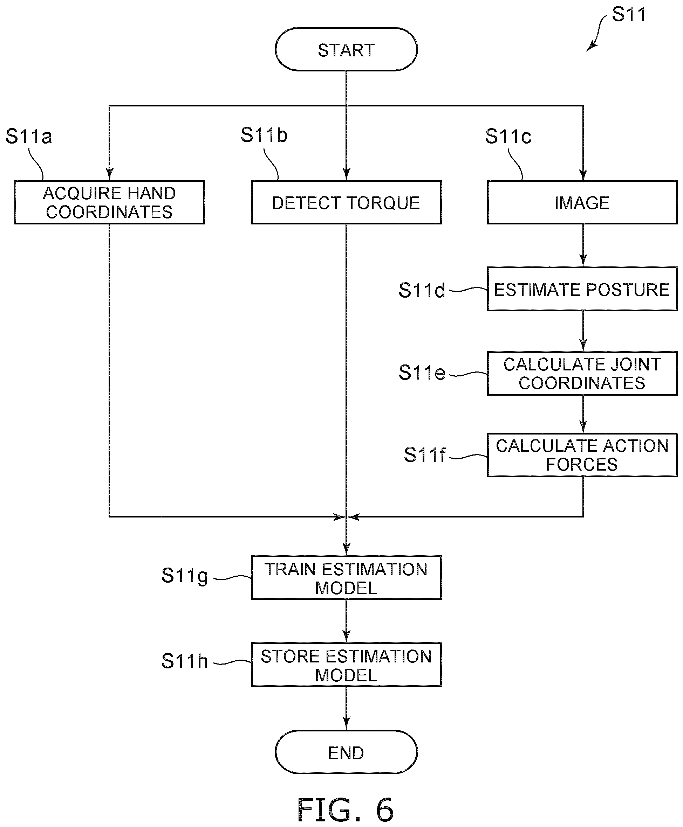

FIG. 6 is a flowchart showing a training method of the estimation model.

When the task is started by the worker W, the MR device 100 performs hand tracking and acquires the hand coordinates (step S11a). The digital torque wrench 10 detects the torque (step S11b). The imaging device 20 images the worker W performing the task (step S11c). The processing device 30 estimates the posture by using an image acquired by the imaging device 20 (step S11d). Based on the result of the posture estimation, the processing device 30 calculates the coordinates of the joints of the worker W (step S11e). The processing device 30 calculates the action forces of the body parts by performing inverse kinematics calculations and inverse dynamics calculations (step S11f).

Steps S11a, S11b, and S11f prepare multiple data sets at multiple timings. The processing device 30 uses the multiple data sets to train the estimation model M1 (step S11g). The processing device 30 stores the trained estimation model M1 (step S11h).

In the example above, the estimation model M1 is trained using short-term action forces every second. Training may be performed using long-term action forces in addition to or instead of such training. For example, muscle activity levels that are obtained over a period of 2 to 5 seconds are averaged to calculate long-term muscle activity levels. The hand coordinates and the torque also are acquired at some timing in this period. Another estimation model is trained by using data sets of the hand coordinates, torque, and long-term muscle activity levels.

There is a possibility that short-term action forces may lead to instantaneous loads that cause a slipped back (acute lower back pain), etc. There is a possibility that long-term action forces may lead to fatigue that causes tendovaginitis, runner's knee (patellofemoral pain syndrome), tennis elbow (lateral epicondylitis), etc.

Training of the danger determination model and training of the proficiency determination model are performed separately from the training of the estimation model of the muscle activity level described above.

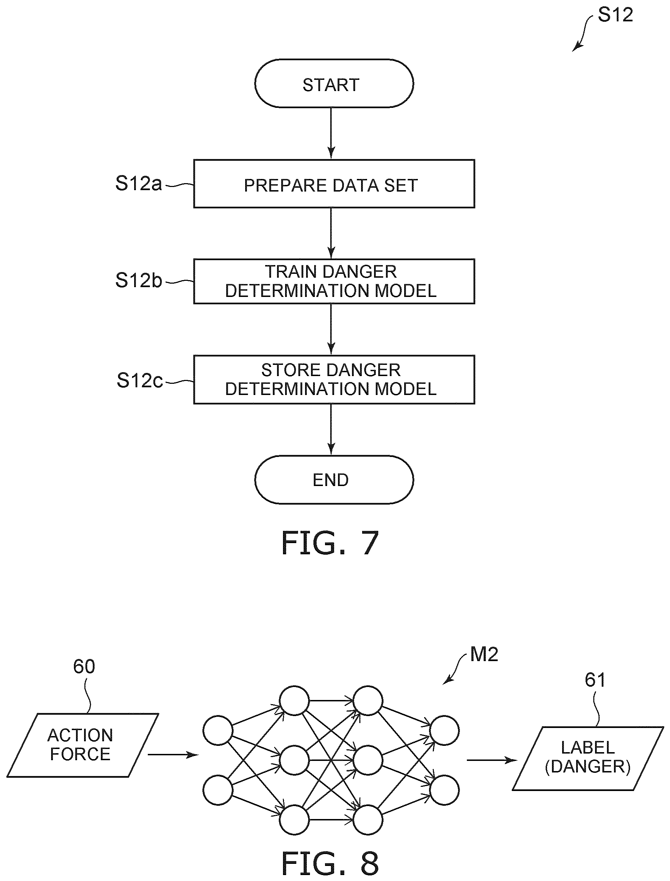

FIG. 7 is a flowchart showing a training method of the danger determination model. FIG. 8 is a schematic view showing the training method of the danger determination model.

First, the data sets to be used to train the danger determination model are prepared (step S12a). The data sets include the action forces and labels for the action forces. The labels indicate the presence of danger for the action forces. The data sets may include the action forces obtained in step S11f and the action forces output from the trained estimation model.

For example, when the muscle activity level of a specific part is large and there is a possibility of injuring a joint or muscle, a label that indicates the danger is assigned. When the muscle activity level is small and there is no possibility of injuring a joint or muscle, a label that indicates no danger is assigned.

Multiple labels may be prepared to indicate the type of danger. For example, multiple labels that indicate specific dangers such as slipped back (acute lower back pain), tendovaginitis, runner's knee, tennis elbow, etc., may be prepared.

The labels and the action forces corresponding to the labels can be prepared using a musculoskeletal simulation. For example, the processing device 30 simulates the motion when there is a danger of slipped back. The processing device 30 acquires the action forces of the body parts at this time. As a result, a data set of the label (slipped back) and the action forces of the parts is obtained. Similarly, data sets that use musculoskeletal simulations are prepared for other types of dangers as well.

As shown in FIG. 8, the processing device 30 uses the action force 60 as input data and uses a label 61 as output data to perform supervised learning of a danger determination model M2 (step S12b of FIG. 7). As a result, the danger determination model is trained to output determination results of the presence of dangers according to the input of the action forces. To increase the determination accuracy, it is favorable for the danger determination model to include a neural network. The processing device 30 stores the trained danger determination model M2 (step S12c).

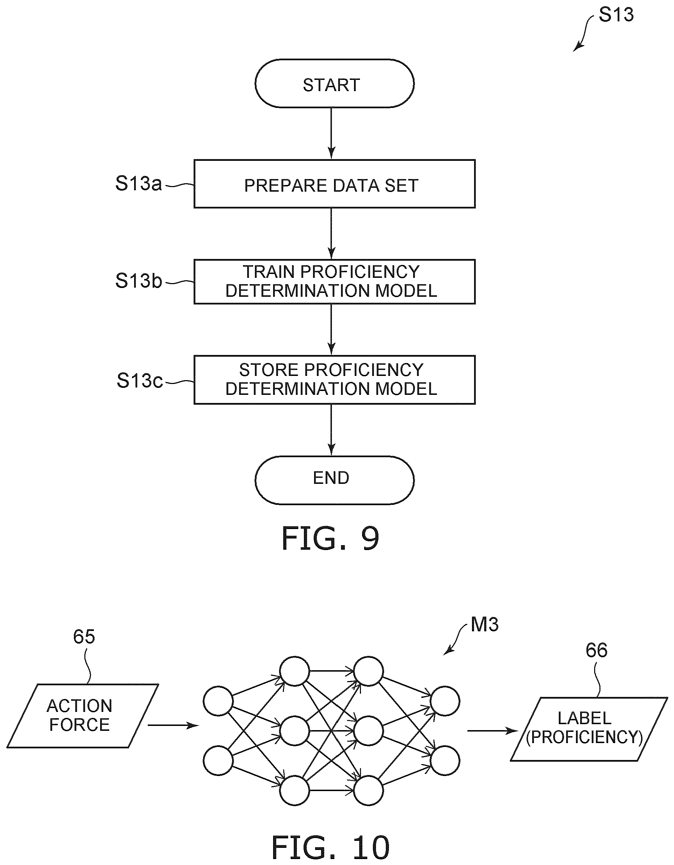

FIG. 9 is a flowchart showing a training method of the proficiency determination model. FIG. 10 is a schematic view showing the training method of the proficiency determination model.

First, data sets that are used to train the proficiency determination model are prepared (step S13a). The data sets include the action forces of the body parts and labels for the action forces. The labels indicate the proficiency of the task for the action forces. For example, the proficiency is represented by a numerical value; and the skill of the task increases as the numerical value increases.

As an example, the proficiency is evaluated based on the time necessary for the task. A person that has a relatively short task time is evaluated as "expert". A person that has a relatively long task time is evaluated as "beginner". The action forces of the workers performing the task are acquired. The labels (proficiency) and the action forces corresponding to the labels are acquired thereby.

As shown in FIG. 10, the processing device 30 uses an action force 65 as input data and uses a label 66 as output data to perform supervised learning of a proficiency determination model M3 (step S13b of FIG. 9). As a result, the proficiency determination model is trained to output the determination result of the proficiency of the worker according to the input of the action forces of the parts. To increase the determination accuracy, it is favorable for the proficiency determination model to include a neural network. The processing device 30 stores the trained proficiency determination model M3 (step S13c).

The estimation model of the action force, the danger determination model, and the proficiency determination model are prepared by the processing described above.

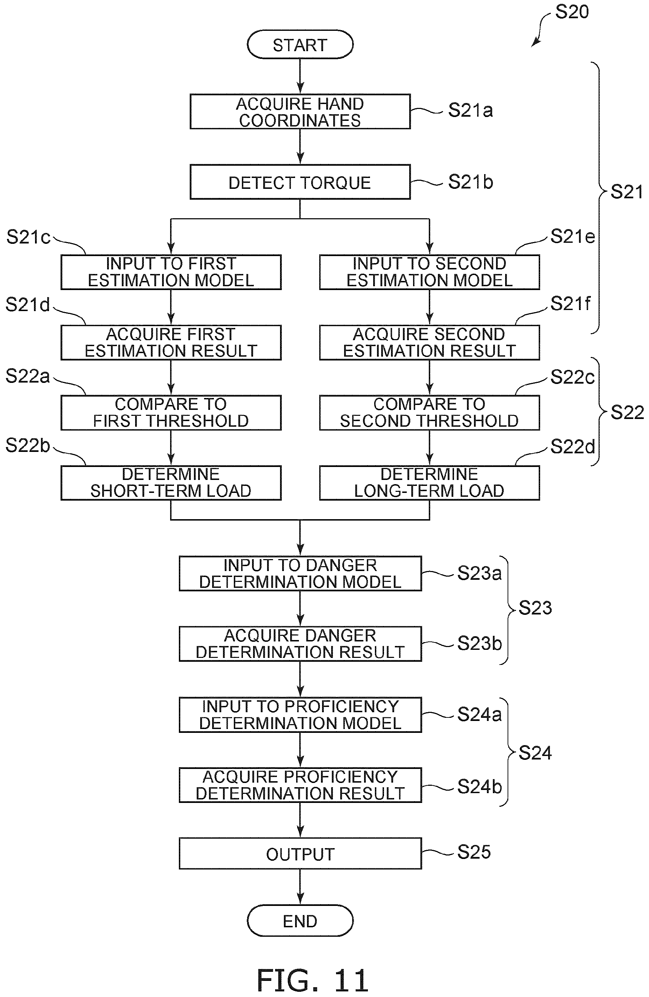

FIG. 11 is a flowchart showing a determination method according to the embodiment.

When the worker W starts the task, the hand coordinates are acquired (step S21a), and the torque is detected (step S21b). The hand coordinates may be calculated using an image of the imaging device 20, or may be acquired using the hand tracking result of the MR device 100. The calculation of the hand coordinates and the detection of the torque are repeated during the task of the worker W.

Favorably, the hand tracking result of the MR device 100 is used. When the worker wears the MR device 100, the MR device 100 is positioned proximate to the hands of the worker W. When viewed from the MR device 100, the hands are not easily concealed by a shadow; and compared to the imaging device 20, the hands are easily detected. By using the hand tracking result, the accuracy of the hand coordinates can be increased.

The processing device 30 inputs the hand coordinates and the torque at any time to the trained estimation model. The estimation model M1 outputs the action force according to the input of the hand coordinates and the torque. The processing device 30 acquires the action force output from the estimation model M1.

As one specific example, a first estimation model that estimates the short-term action force is prepared, and a second estimation model that estimates the long-term action force is prepared. The first estimation model is trained using a data set every second as described with reference to FIG. 3. The second estimation model is trained using a data set every 2 to 5 seconds.

The processing device 30 inputs the hand coordinates and the torque to the first estimation model (step S21c). The processing device 30 acquires a first estimation result output from the first estimation model (step S21d). The processing device 30 inputs the hand coordinates and the torque to the second estimation model (step S21e). The processing device 30 acquires a second estimation result output from the second estimation model (step S21f).

The processing device 30 compares the first estimation result output from the first estimation model to a preset first threshold (step S22a). For example, one or more thresholds are preset for the first estimation result. The processing device 30 determines the short-term load on the worker W based on the comparison result between the first estimation result and the threshold (step S22b).

The processing device 30 compares the second estimation result output from the second estimation model to a preset second threshold (step S22c). For example, one or more thresholds are preset for the second estimation result. The processing device 30 determines the long-term load on the worker W based on the comparison result between the second estimation result and the threshold (step S22d).

Then, the processing device 30 inputs the action force acquired in step S21d to the danger determination model (step S23a). The processing device 30 acquires the determination result of the danger output from the danger determination model (step S23b). The danger determination model is used to find dangers that are difficult to determine using only the comparison between the action force and the threshold. By using the danger determination model to determine the presence of dangers, the risk of bodily injury of the worker can be more accurately determined.

The processing device 30 inputs, to the proficiency determination model, the action force acquired in step S21d (step S24a). The processing device 30 acquires the determination result of the proficiency output from the proficiency determination model (step S24b). The processing device 30 outputs the obtained result (step S25).

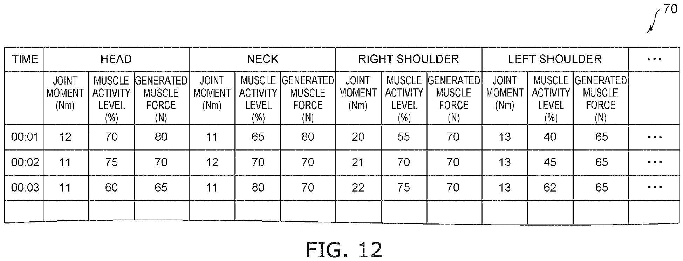

FIG. 12 is a table illustrating an output result of the first estimation model.

For example, in the task, the hand coordinates and the torque are acquired every second such as those shown in FIG. 3. The processing device 30 obtains an estimation result 70 shown in FIG. 12 by inputting the hand coordinates and the torque to the first estimation model. In the example shown in FIG. 12, the joint moment, the muscle activity level, and the generated muscle force are estimated every second for each body part.

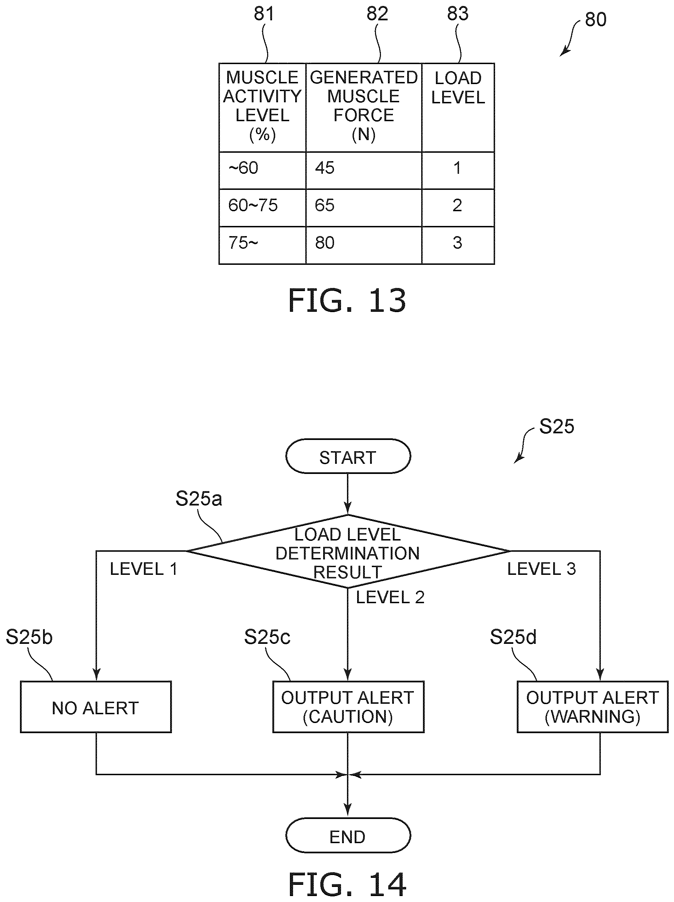

FIG. 13 is a table illustrating thresholds for determining the load.

The estimation results shown in FIG. 12 are compared to the thresholds shown in FIG. 13. In the table 80 shown in FIG. 13, multiple thresholds are set in a column 81 of the muscle activity level and a column 82 of the generated muscle force. The muscle activity level and the generated muscle force of each part included in the estimation result are compared to the thresholds defined by the columns 81 and 82. The load level that is defined by a column 83 is determined according to the comparison result. The data that is shown in FIGS. 12 and 13 is used to determine the load level for each body part every second.

When the second estimation model is used, the estimation result from the second estimation model also is obtained. A threshold is set for the estimation result of the second estimation model. By comparing the estimation result obtained from the second estimation model to the threshold, the processing device 30 determines the long-term load level on each body part.

The processing device 30 also acquires the determination result of the presence of danger and the determination result of the proficiency of the worker by inputting the action force output from the estimation model to the danger determination model and the proficiency determination model.

It is desirable to transmit the results to the worker when it is determined that there is a load or danger based on the determination result. For example, based on the determination result, an alert is displayed in the MR device 100 or output as a voice from the MR device 100.

The presence of the load may be determined based on the action force, or the load may be classified into three or more load levels as shown in FIG. 13. The content of the output may be different according to the classification result of the load level. Similarly, the presence of danger may be determined based on the action force, or the danger may be classified into three or more danger levels. The content of the output may be different according to the classification result of the danger level.

FIG. 14 is a flowchart illustrating a control of an output corresponding to the determination result of the load.

For example, the processing device 30 may perform the processing shown in FIG. 14 in step S25 shown in FIG. 11. The processing device 30 refers to the determination result of the load (step S25a). When the load level is "1", the processing device 30 does not output an alert (step S25b). The load level being 1 means that there is substantially no load. When the load level is "2", the processing device 30 outputs an alert urging caution (step S25c). When the load level is "3", the processing device 30 outputs a warning alert (step S25d). For example, compared to step S25c, an alert together with a stronger warning color are displayed in step S25d. Compared to step S25c, a larger alert may be displayed in step S25d. Compared to step S25c, the alert may be output as a louder voice in step S25d.

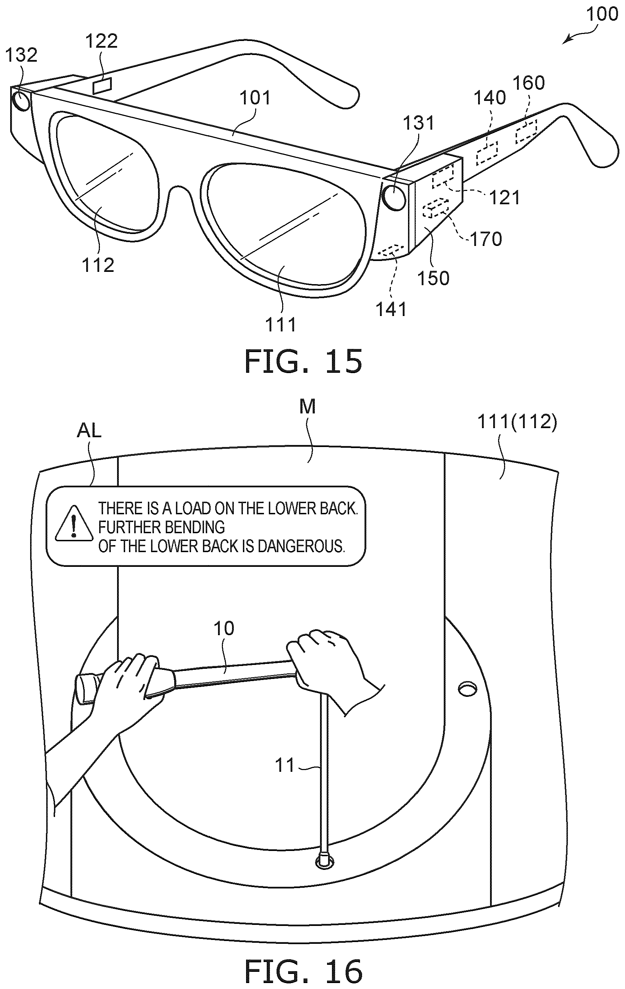

FIG. 15 is a schematic view showing an example of a cross reality device according to the embodiment.

FIG. 15 shows a MR device as an example of the cross reality device. As shown in FIG. 15, the MR device 100 includes a frame 101, a lens 111, a lens 112, a projection device 121, a projection device 122, an image camera 131, a depth camera 132, a sensor 140, a microphone 141, a processing device 150, a battery 160, and a storage device 170.

In the illustrated example, the MR device 100 is a binocular head mounted display. Two lenses, i.e., the lens 111 and the lens 112, are fit into the frame 101. The projection device 121 and the projection device 122 respectively project information onto the lenses 111 and 112.

The projection device 121 and the projection device 122 display a recognition result of a body of a worker, a virtual object, etc., on the lenses 111 and 112. Only one of the projection device 121 or the projection device 122 may be included; and information may be displayed on only one of the lens 111 or the lens 112.

The lens 111 and the lens 112 are light-transmissive. The worker can visually recognize reality via the lenses 111 and 112. Also, the worker can visually recognize the information projected onto the lenses 111 and 112 by the projection devices 121 and 122. Information is displayed to overlap real space by being projected by the projection devices 121 and 122.

The image camera 131 detects visible light and obtains a two-dimensional image. The depth camera 132 irradiates infrared light and obtains a depth image based on the reflected infrared light. The sensor 140 is a six-axis detection sensor and is configured to detect angular velocities in three axes and accelerations in three axes. The microphone 141 accepts an audio input.

The processing device 150 controls components of the MR device 100. For example, the processing device 150 controls the display by the projection devices 121 and 122. The processing device 150 detects movement of the visual field based on a detection result of the sensor 140. The processing device 150 modifies the display by the projection devices 121 and 122 according to the movement of the visual field. The processing device 150 also is configured to perform various processing by using data obtained from the image camera 131 and the depth camera 132, data of the storage device 170, etc.

The battery 160 supplies power necessary for the operations to the components of the MR device 100. The storage device 170 stores data necessary for the processing of the processing device 150, data obtained by the processing of the processing device 150, etc. The storage device 170 may be located outside the MR device 100, and may communicate with the processing device 150.

The MR device according to the embodiment is not limited to the illustrated example, and may be a monocular head mounted display. The MR device may be an eyeglasses-type as illustrated, or may be a helmet-type.

The processing device 150 may function as the processing device 30. In such a case, the processing device 30 may be omitted from the determination system 1 shown in FIG. 2. Or, a portion of the processing performed by the processing device 30 according to the method described above may be performed by the processing device 150.

FIG. 16 is a schematic view showing an output example of the determination system according to the embodiment.

In the example shown in FIG. 16, the worker W is using the digital torque wrench 10 and an extension bar 11 to tighten a screw at the member M. In the task, the processing device 150 performs hand tracking by using the imaging results of the image camera 131 and the depth camera 132. The hands of the worker W are recognized by the hand tracking; and the hand coordinates are calculated.

The processing device 30 receives the detected value of the torque from the digital torque wrench 10. The processing device 30 inputs the hand coordinates and the detected value of the torque to an estimation model that acquires the estimation result of the action force. The processing device 30 uses the estimation result to determine the load. The processing device 30 uses a danger determination model and a proficiency determination model to determine the danger and determine the proficiency.

In the example shown in FIG. 16, based on the estimation result of the action force, it is determined that a load is applied to the lower back of the worker and there is a danger to the lower back. The processing device 30 transmits the determination results to the MR device 100. The processing device 150 displays an alert AL indicating that a load is applied to the lower back and there is a danger to the lower back.

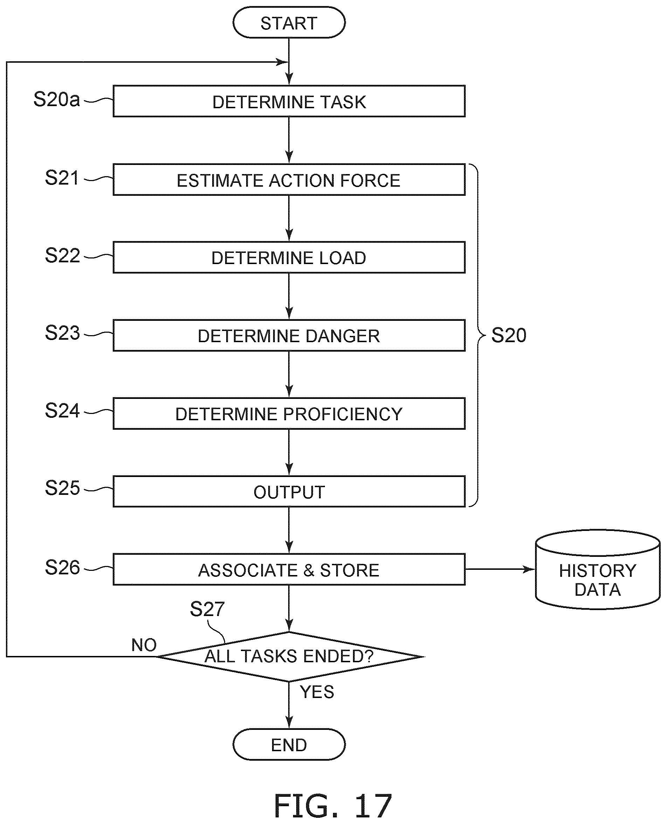

FIG. 17 is a flowchart showing another determination method according to the embodiment.

The determination result that is obtained by the determination method may be stored by being associated with task data. For example, as shown in FIG. 17, first, the processing device 30 determines the task to be performed (step S20a). For example, the worker inputs, to the processing device 30, the task to be performed. The processing device 30 determines the task to be performed by accepting the input. The processing device 30 may determine the task to be performed based on the time and a preregistered schedule. The processing device 30 may determine the task to be performed based on an image that is imaged by the imaging device 20 or the image camera 131. For example, the members in the image are compared to a template image that is prepared beforehand. Each template image is associated with one of the tasks. The processing device 30 uses template matching to determine the template image most similar to the members in the image. The processing device 30 determines the task associated with the template image as the task to be performed.

The processing device 30 estimates the action force (step S21), determines the load (step S22), determines the danger (step S23), and determines the proficiency (step S24). The processing device 30 outputs the determination results (step S25). The processing device 30 also associates the determination results with data of the task determined in step S20a and stores the determination results as history data (step S26).

The processing device 30 determines whether or not all of the tasks have ended (step S27). When not all tasks have ended, step S20a is re-performed. As a result, the multiple determination results obtained in step S20 are associated with the data of the tasks.

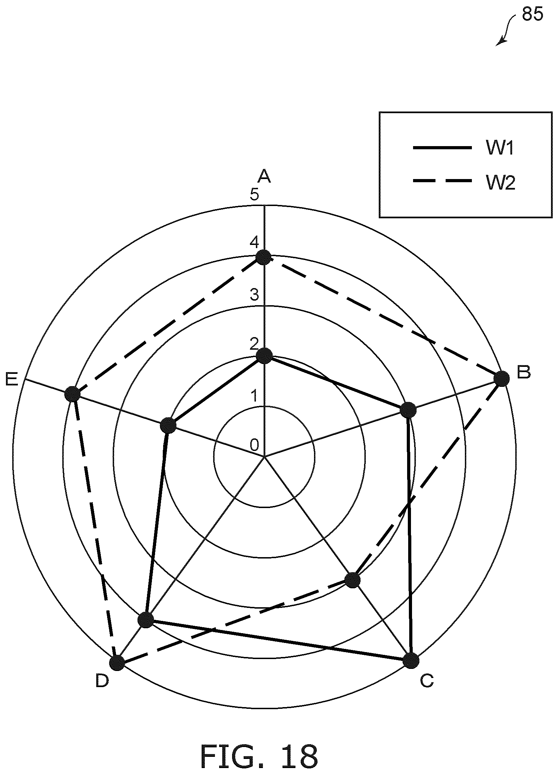

FIG. 18 is a schematic view showing another output example of the determination system according to the embodiment.

In the example shown in FIG. 18, the proficiencies of the workers are displayed in a radar chart 85 for each process. For example, workers W1 and W2 perform processes A to E. As a result, the proficiency of the worker W1 and the proficiency of the worker W2 are determined for each of the processes A to E. In the illustrated example, the proficiency is evaluated using the six levels of "0" to "5". The proficiency increases as the numerical value increases.

As shown in FIG. 18, by displaying the proficiency for each task for each worker, the tasks at which the workers are skillful and the tasks at which the workers are inept can be easily ascertained. For example, for tasks determined to have a low proficiency, the work efficiency of a beginner can be increased by an expert instructing the beginner. For tasks determined to have high proficiency, the proficiencies of the workers can be improved by sharing video images of such tasks among multiple workers.

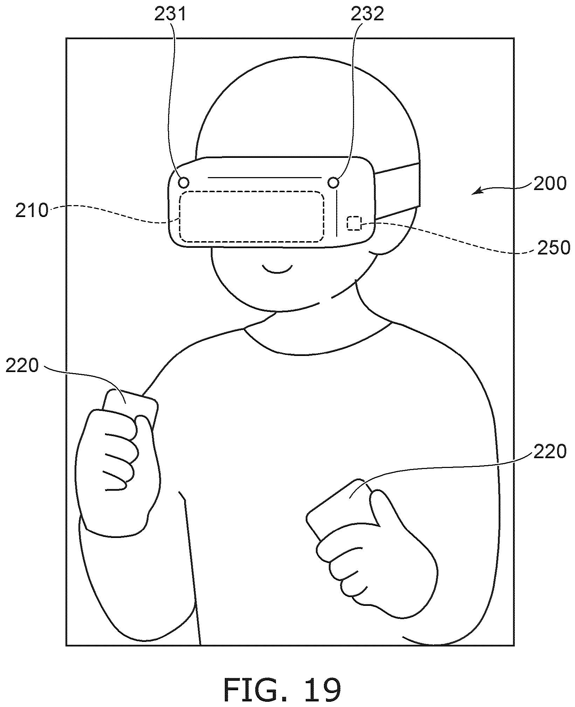

FIG. 19 is a schematic view showing another example of the cross reality device according to the embodiment.

FIG. 19 shows a VR device as an example of the cross reality device. The VR device 200 shown in FIG. 19 is a goggle-type. The VR device 200 includes a monitor 210, a remote control 220, an image camera 231, a depth camera 232, and a processing device 250. A virtual space is displayed by the monitor 210. The wearer views the virtual space displayed in the monitor 210.

The wearer grasps the remote control 220 and operates the remote control 220. The remote control 220 includes at least one selected from the group consisting of an acceleration sensor and an angular velocity sensor. The remote control 220 is an example of a device that includes a sensor. When the remote control 220 is operated, a signal is transmitted from the remote control 220 to the processing device 250. For example, the wearer of the VR device 200 can play a video game with the VR device 200 while operating the remote control 220.

The image camera 231 and the depth camera 232 image frontward of the wearer. The processing device 250 uses the imaging result to perform hand tracking. As a result, the hand coordinates of the wearer are calculated.

The processing device 30 (or the processing device 250) performs the determination shown in FIG. 1 (step S20) by using the hand coordinates and the detected value (the acceleration or the angular velocity) of the remote control 220. The load, danger, and proficiency of the wearer are determined thereby.

Advantages of an aspect of the embodiment will now be described.

Forces act on the body of a person during the motion of the person. For example, a force is generated by a muscle; and that force applies a force to a joint. There is a possibility that an action force of the body may apply a load to the body unintentionally or unknowingly, and the body may be hurt or injured. According to an embodiment of the invention, to prevent bodily injury, the action force of the body is used to determine the load on the person or the danger to the person. When determining a load or danger, if a load or danger is determined to be present, bodily injury can be avoided by stopping or improving the motion. The action force is obtained by inputting, to the estimation model, the hand coordinates and the detected value of the sensor of the device. It is therefore unnecessary to prepare various sensors to estimate the action force. For example, the data necessary to estimate the action force can be obtained without obstructing the motion of the person.

Advantages of another aspect of the embodiment will now be described.

When a person performs a specific motion, there are cases where the motion requires accuracy or efficiency. For example, the efficiency of the task can be increased by making the motion faster or more accurate during the task. When playing a game, the game can be played skillfully when the motions are accurate. According to an embodiment of the invention, the action force is used to determine the proficiency of the person. As described above, the action force is estimated using the hand coordinates and the detected value of a sensor. Therefore, according to the embodiment, the proficiency can be more easily determined.

According to the embodiment of the invention, the load on the person, the danger to the person, or the proficiency of the person can be more easily determined. According to the embodiment, the data that is necessary to determine the load, the danger, or the proficiency can be acquired without obstructing the motion of the person.

The embodiment is particularly favorable to determine tasks that are repeatedly performed. For example, many screws are tightened when producing a product. As an example, in the production of a large product such as a generator, a plant, etc., screws are tightened at thousands of locations for one product. A person that performs the assembly task of the product repeatedly tightens screws. Therefore, the load on the body is extremely large. Furthermore, various regulations exist for the sequence of the screw-tightening, the strength of the screw-tightening, etc.; and it is necessary to perform the tasks according to the regulations. Therefore, the progress of the task differs drastically according to the proficiency of the worker. For example, in order for the tasks to proceed according to the task schedule, it is necessary to more accurately ascertain the proficiencies of the workers.

According to the embodiment, the load on the body or the danger can be easily determined. When a load or danger is determined to be present, the alert AL is output as shown in FIG. 16. Bodily injury can be avoided by urging the worker to improve the posture. Or, according to the embodiment, the proficiency can be easily determined. By representing the proficiency as data, persons can be more appropriately assigned to the tasks; and the accuracy of the progress of the task schedule can be increased. Personnel training can be more efficiently performed by educating the workers based on the proficiency determination results.

According to the embodiment, a model that includes a neural network is trained using the hand coordinates, the detected value of a sensor, and the action force. For example, the hand coordinates can be easily acquired using a camera of a cross reality device. The detected value can be easily acquired by a device that includes a sensor. The acquisition of such data does not easily obstruct human motion. According to the trained model, the action force can be estimated using such data. For example, as described above, the load on the person, the danger to the person, or the proficiency of the person can be determined using the estimated action force.

An example is described in the embodiments above in which the action force is used to determine three things: the load on the person, the danger to the person, and the proficiency of the person. Embodiments are not limited to the example. For example, only one or two selected from the group consisting of the load on the person, the danger to the person, and the proficiency of the person may be determined.

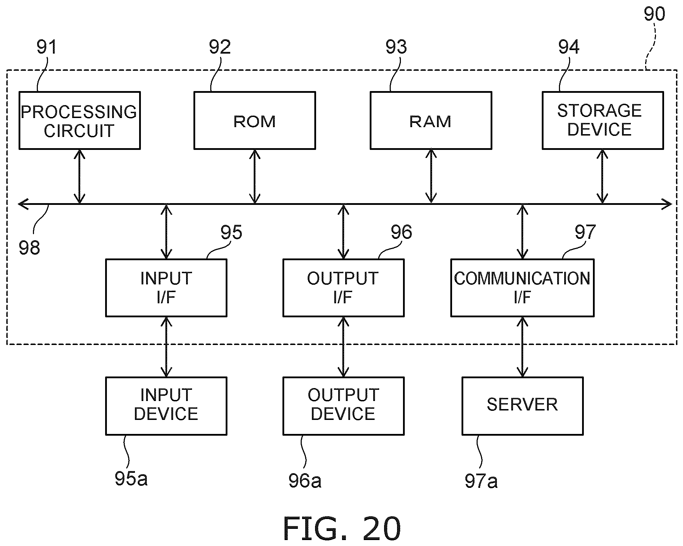

FIG. 20 is a schematic view illustrating a hardware configuration.

For example, the processing device 30, 150, or 250 includes a computer 90 shown in FIG. 20. The computer 90 includes a processing circuit 91, ROM 92, RAM 93, a storage device 94, an input interface 95, an output interface 96, and a communication interface 97.

The ROM 92 stores programs controlling operations of the computer 90. The ROM 92 stores programs necessary for causing the computer 90 to realize the processing described above. The RAM 93 functions as a memory region into which the programs stored in the ROM 92 are loaded.

The processing circuit 91 includes an arithmetic processor such as a CPU, a GPU, etc. The processing circuit 91 uses the RAM 93 as work memory to execute the programs stored in at least one of the ROM 92 or the storage device 94. When executing the programs, the processing circuit 91 executes various processing by controlling configurations via a system bus 98.

The storage device 94 stores data necessary for executing the programs and/or data obtained by executing the programs.

The input interface (I/F) 95 can connect the computer 90 and an input device 95a. The input I/F 95 is, for example, a serial bus interface such as USB, etc. The processing circuit 91 can read various data from the input device 95a via the input I/F 95.

The output interface (I/F) 96 can connect the computer 90 and an output device 96a. The output I/F 96 is, for example, an image output interface such as Digital Visual Interface (DVI), High-Definition Multimedia Interface (HDMI (registered trademark)), etc. The processing circuit 91 can transmit data to the output device 96a via the output I/F 96 and cause the output device 96a to display an image.

The communication interface (I/F) 97 can connect the computer 90 and a server 97a outside the computer 90. The communication I/F 97 is, for example, a network card such as a LAN card, etc. The processing circuit 91 can read various data from the server 97a via the communication I/F 97.

The storage device 94 includes at least one selected from a hard disk drive (HDD) and a solid state drive (SSD). The input device 95a includes at least one selected from a mouse, a keyboard, a microphone (audio input), and a touchpad. The output device 96a includes at least one selected from a monitor, a projector, a printer, and a speaker. A device such as a touch panel that functions as both the input device 95a and the output device 96a may be used.

The processing necessary for the determination method may be performed by one computer 90 or may be performed by collaboration by multiple computers 90.

The processing of the various data described above may be recorded, as a program that can be executed by a computer, in a magnetic disk (a flexible disk, a hard disk, etc.), an optical disk (CD-ROM, CD-R, CD-RW, DVD-ROM, DVD±R, DVD±RW, etc.), semiconductor memory, or another non-transitory computer-readable storage medium.

For example, the data of the recording medium is read by a computer (a processing circuit). The recording format (the storage format) of the recording medium is arbitrary. For example, the computer reads a program from the recording medium and causes a CPU to execute instructions based on the program. The computer may acquire (or read) the program via a network.

Embodiments of the invention may be realized as a determination device that causes a processing device to execute the program of the determination method described above. The determination device can determine the load on the person, the danger to the person, or the proficiency of the person. Embodiments of the invention also may be realized as a training device that causes a processing device to execute the program of the training method described above. The training device trains a model that includes a neural network by using the coordinates and the detected value as input data and by using the action force as output data.

Embodiments of the invention include the following features.

### Feature 1

A determination method, including:
causing a processing device to
measure coordinates of a hand of a person based on an image in which the hand is visible,
receive a detected value from a sensor of a device held by the person,
input the coordinates and the detected value to an estimation model configured to estimate an action force of a body,
determine at least one selected from the group consisting of a load on the person, a danger to the person, and a proficiency of the person by using an action force of a body of the person output from the estimation model.

### Feature 2

The determination method according to feature 1, in which
the processing device is caused to:
determine the load by comparing the action force to a threshold, the threshold being preset, and
output an alert when the load is determined to be present.

### Feature 3

The determination method according to feature 1 or 2, in which
the processing device is caused to:
determine the danger to the person by inputting the action force to a danger determination model configured to determine a danger; and
output an alert when the danger to the person is determined to be present.

### Feature 4

The determination method according to any one of features 1 to 3, in which
the processing device is caused to:
determine the proficiency of the person by inputting the action force to a proficiency determination model configured to determine a proficiency; and
output the proficiency that is determined.

### Feature 5

The determination method according to any one of features 1 to 4, in which
the action force is at least one selected from the group consisting of a muscle activity level, a generated muscle force, an antagonistic muscle force, a joint force, and a joint moment.

### Feature 6

A determination device, including:
a processing device,
the determination device being configured to cause the processing device to perform the determination method according to any one of features 1 to 5.

### Feature 7

A determination system, including:
the determination device according to feature 6;
an imaging device configured to image a hand of a person; and
the device held by the person.

### Feature 8

The determination system according to feature 7, in which
the sensor detects at least one selected from the group consisting of a torque, an acceleration, and an angular velocity.

### Feature 9

A cross reality device, including:
a processing circuit; and
an imaging device configured to image a hand of a person,
the cross reality device being configured to cause the processing circuit to perform the determination method according to any one of features 1 to 5.

### Feature 10

A program,
the program, when executed by a processing device, causing the processing device to perform the determination method according to any one of features 1 to 5.

### Feature 11

A training method, including:
causing a processing device to
acquire coordinates of a hand of a person holding a device, a detected value of a sensor of the device, and an action force of a body of the person, and
train a model including a neural network by using the coordinates and the detected value as input data and by using the action force as output data.

### Feature 12

A training device, including:
a processing circuit,
the training device causing a processing circuit to perform the training method according to feature 11.

### Feature 13

A program,
the program, when executed by a processing device, causing the processing device to perform the training method according to feature 11.

### Feature 14

A storage medium storing the program according to feature 10 or 13.

According to the embodiments above, a determination method, a determination device, a determination system, a cross reality device, a program, and a storage medium are provided in which a load on a person, a danger to the person, or a proficiency of the person can be more easily determined. A training method, a training device, a program, and a storage medium also are provided, and can train a model to estimate an action force of the body of the person by using data that is easy to acquire.

In the specification, "or" means that "at least one" of the items listed in the text can be employed.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the invention. Moreover, above-mentioned embodiments can be combined mutually and can be carried out.

## Claims

1. A determination method, comprising:
causing a processing device to
measure coordinates of a hand of a person based on an image in which the hand is visible,
receive a detected value from a sensor of a device held by the person,
input the coordinates and the detected value to an estimation model configured to estimate an action force of a body,
determine at least one selected from the group consisting of a load on the person, a danger to the person, and a proficiency of the person by using an action force of a body of the person output from the estimation model.

2. The determination method according to claim 1, wherein
the processing device is caused to:
determine the load by comparing the action force to a threshold, the threshold being preset, and
output an alert when the load is determined to be present.

3. The determination method according to claim 1 or 2, wherein
the processing device is caused to:
determine the danger to the person by inputting the action force to a danger determination model configured to determine a danger; and
output an alert when the danger to the person is determined to be present.

4. The determination method according to any one of claims 1 to 3, wherein
the processing device is caused to:
determine the proficiency of the person by inputting the action force to a proficiency determination model configured to determine a proficiency; and
output the proficiency that is determined.

5. The determination method according to any one of claims 1 to 4, wherein
the action force is at least one selected from the group consisting of a muscle activity level, a generated muscle force, an antagonistic muscle force, a joint force, and a joint moment.

6. A determination device, comprising:
a processing device,
the determination device being configured to cause the processing device to perform the determination method according to any one of claims 1 to 5.

7. A determination system, comprising:
the determination device according to claim 6;
an imaging device configured to image a hand of a person; and
the device held by the person.

8. The determination system according to claim 7, wherein
the sensor detects at least one selected from the group consisting of a torque, an acceleration, and an angular velocity.

9. A cross reality device, comprising:
a processing circuit; and
an imaging device configured to image a hand of a person,
the cross reality device being configured to cause the processing circuit to perform the determination method according to any one of claims 1 to 5.

10. A program,
the program, when executed by a processing device, causing the processing device to perform the determination method according to any one of claims 1 to 5.

11. A training method, comprising:
causing a processing device to
acquire coordinates of a hand of a person holding a device, a detected value of a sensor of the device, and an action force of a body of the person, and
train a model including a neural network by using the coordinates and the detected value as input data and by using the action force as output data.

12. A training device, comprising:
a processing circuit,
the training device causing a processing circuit to perform the training method according to claim 11.

13. A program,
the program, when executed by a processing device, causing the processing device to perform the training method according to claim 11.

14. A storage medium storing the program according to claim 10 or 13.
